# EUROPEAN PATENT APPLICATION

(11) **EP 3 945 099 A1**
(43) Date of publication of application: **02.02.2022**
(21) Application number: 20188786.6
(22) Date of filing: 30.07.2020
(51) Int. Cl.: C08F 6/06

(54) **PROCESS FOR REMOVAL OF FLUOROORGANIC COMPOUNDS FROM AQUEOUS MEDIA**

(71) Applicant: 3M Innovative Properties Company, Saint Paul, MN 55133-3427 (US)
(72) Inventor: Hintzer, Klaus, 84556 Kastl (DE); Koenigsmann, Herbert, 84508 Burgkirchen (DE); Huber, Stefan, 84180 Loiching (DE); Krupp, Valentina, 84478 Waldkraiburg (DE); Schmick, Scott, Lake Elmo, MN 55042 (US); Zhang, Zhongxing, Woodbury, MN 55125 (US); Hirschberg, Markus, 84453 Mühldorf (DE); Lamanna, Bill, Stillwater, MN 55082 (US); Barrera, Mike, Oakdale, MN 55128 (US)
(74) Representative: Hettstedt, Stephan

(57) **Abstract**

The present disclosure provides a process for removing fluoroorganic acidic compounds from a solution comprising at least one protic solvent, comprising the following steps:
(i) forming a mixture of
a. a solution comprising at least one fluoroorganic acidic compound comprising less than 10 carbon atoms and at least one protic solvent, wherein the solution has a pH-value of up to 6, with
b. an extraction composition comprising at least one alkylamine and at least one organic solvent;

(ii) reacting the fluoroorganic acidic compound with the alkylamine to form a hydrophobic ionic compound comprising the anion of the fluoroorganic acidic compound and the cation of the alkyl amine;
(iii) separating the mixture into a first phase comprising the at least one protic solvent and no greater than 50% by weight of the total amount of the at least one fluoroorganic acidic compound initially present in the solution in step (i); and a second phase comprising the at least one organic solvent and the hydrophobic ionic compound;
(iv) removing the second phase from the first phase; and
(v) repeating steps (i) to (iv) at least a second time, wherein the first phase obtained in step (iv) is used as the solution in step (i).

## Description

### Field

The present disclosure relates to a continuous process for removing fluoroorganic compounds from aqueous media by means of extraction with alkylamines in organic solvents.

### Background

Fluorinated compositions have been used in a wide variety of applications including fluorochemicals for: water-proofing materials, fire-fighting foams for electrical and grease fires, semi-conductor etching, and lubricants; and fluoropolymers for: hoses, gaskets, seals, coatings, and films. Reasons for such widespread use of fluorinated compositions include their favorable physical properties, which include chemical inertness, low coefficients of friction, and low polarizabilities (i.e., fluorophilicity).

After production of a fluorinated composition, fluorinated compounds, including, for example, starting materials and reaction by-products, may be removed from the waste streams generated by the production. The removal of the fluorinated compounds may be to recover expensive starting material and/or to avoid undesirable release of the fluorinated compounds into the environment.

Processes to remove or recover fluorinated compounds include ion exchange, ultrafiltration, distillation, liquid-liquid extraction, reverse osmosis, and adsorption on clays, carbon and other media. Such processes have been described in U.S. Publ. Nos. 2006/0205828 (Maurer et al.), 2007/0025902 (Hintzer et al), and 2010/0084343 (Mader et al.), and U.S. Pat. Nos. 3,882,153 (Seki et al), 4,369,266 (Kuhls, et al.), 6,642,415 (Fuhrer et al), 7,279,522 (Dadalas et al), and 5,603,812 (Hommeltoft). It is known that these processes may be associated with deficiencies of various degrees, such as low efficiency, high investment costs, or an increased environmental burden. Another more recent effort comprised the removal of fluoroorganic acids from aqueous media with alkylammonium compounds. However, providing of efficient and environmentally benign methods for removal of fluoroorganic compounds remains a desire in the art, in particular for large-scale or industrial operations.

### Summary

The present disclosure provides a process for removing fluoroorganic compounds from a solution comprising at least one protic solvent, comprising the following steps:
(i) forming a mixture of
   a. a solution comprising at least one fluoroorganic acidic compound comprising less than 10 carbon atoms and at least one protic solvent, wherein the solution has a pH-value of up to 6, with
   b. an extraction composition comprising at least one alkylamine and at least one organic solvent;
(ii) reacting the fluoroorganic acidic compound with the alkylamine to form a hydrophobic ionic compound comprising the anion of the fluoroorganic acidic compound and the cation of the alkyl amine;
(iii) separating the mixture into a first phase comprising the at least one protic solvent and no greater than 50% by weight of the total amount of the at least one fluoroorganic compound initially present in the solution in step (i); and a second phase comprising the at least one organic solvent and the hydrophobic ionic compound;
(iv) removing the second phase from the first phase; and
(v) repeating steps (i) to (iv) at least a second time, wherein the first phase obtained in step (iv) is used as the solution in step (i).

### Detailed Description

Before any embodiments of this disclosure are explained in detail, it is to be understood that the disclosure is not limited in its application to the details of construction and the arrangement of components set forth in the following description. The invention is capable of other embodiments and of being practiced or of being carried out in various ways. As used herein, the term "a", "an", and "the" are used interchangeably and mean one or more; and "and/or" is used to indicate one or both stated cases may occur, for example A and/or B includes, (A and B) and (A or B). Also herein, recitation of ranges by endpoints includes all numbers subsumed within that range (e.g., 1 to 10 includes 1.4, 1.9, 2.33, 5.75, 9.98, etc.). Also herein, recitation of "at least one" includes all numbers of one and greater (e.g., at least 2, at least 4, at least 6, at least 8, at least 10, at least 25, at least 50, at least 100, etc.). Also, it is to be understood that the phraseology and terminology used herein is for the purpose of description and should not be regarded as limiting. Contrary to the use of "consisting", which is meant to be limiting, the use of "including," "containing", "comprising," or "having" and variations thereof is meant to be not limiting and to encompass the items listed thereafter as well as additional items. It should be noted, however, that the use of "comprising" herein also encompasses the term of "consisting of", i.e. the use of "consisting of" in the sense of "consisting only of" is not excluded in the present disclosure per se.

Amounts of ingredients of a composition may be indicated by % by weight (or "% wt". or "wt.-%") unless specified otherwise. The amounts of all ingredients gives 100 % wt unless specified otherwise. If the amounts of ingredients are identified by % mole the amount of all ingredients gives 100% mole unless specified otherwise.

Unless explicitly indicated, all preferred ranges and embodiments may be combined freely.

Parameters as described herein may be determined as described in detail in the experimental section.

The present disclosure provides a process for removing fluoroorganic acidic compounds from a solution comprising at least one protic solvent, comprising the following steps:
(i) forming a mixture of
   a. a solution comprising at least one fluoroorganic acidic compound comprising less than 10 carbon atoms and at least one protic solvent, wherein the solution has a pH-value of up to 6, with
   b. an extraction composition comprising at least one alkylamine and at least one organic solvent;
(ii) reacting the fluoroorganic acidic compound with the alkylamine to form a hydrophobic ionic compound comprising the anion of the fluoroorganic acidic compound and the cation of the alkyl amine;
(iii) separating the mixture into a first phase comprising the at least one protic solvent and no greater than 50% by weight of the total amount of the at least one fluoroorganic acidic compound initially present in the solution in step (i); and a second phase comprising the at least one organic solvent and the hydrophobic ionic compound;
(iv) removing the second phase from the first phase; and
(v) repeating steps (i) to (iv) at least a second time, wherein the first phase obtained in step (iv) is used as the solution in step (i).

With this process according to the present disclosure, an efficient removal of the fluoroorganic acidic compounds from solutions in protic solvents becomes feasible. The process according to the present disclosure is particularly advantageous for the removal of fluorinated acidic compounds from alcoholic and/or aqueous solvents or solvent mixtures on a large scale or in continuous processes. This is highly advantageous for treatment of large amounts of sewage as necessary during large-scale industrial manufacturing or laboratory sites. In this regard, it is believed that the fluoroorganic acidic compound reacts with the at least one alkylamine to form a hydrophobic ionic compound comprising the fluoroorganic acidic anion and the alkylammonium. .... This hydrophobic ionic compound then readily transfers from the protic solvent of the first solution into the organic solvent of the second solution. Accordingly, when separating the first (protic) phase from the (organic) second phase, an effective removal of a major part of the fluoroorganic acidic compounds initially present in the solution a. is achieved by the process according to the present disclosure.

Thus, in step (i) of the process according to the present disclosure, a mixture is formed of the solution a. and the extraction composition. Forming a mixture includes providing solution a. and extraction composition b., bring both a. and b. into contact with each other, and may further include mixing, stirring, agitating or other steps well-known to the skilled person to form a mixture of two liquid components.

In step (ii), the reaction between the alkylamine and the fluoroorganic acidic compound takes place, in which the hydrophobic ionic compound is formed. In this regard, "reacting" includes "allowing to react", which means the case when both components get into contact, the reaction automatically takes place.

Further, in step (iii) of the process according to the present disclosure, the mixture is separated into a first phase comprising the at least one protic solvent and no greater than 50 wt.-% of the total amount of the fluoroorganic acidic compound initially present in the solution a. in step (i). Given the hydrophobic nature of the ionic compound formed in step (ii) and a generally desirable certain immiscibility of the at least one protic solvent and the at least one organic solvent, a separation of the mixture in the two phases as described herein may readily take place once mixing as described herein is stopped. This may include transfer of the mixture as described herein into another vessel or compartment where no mixing or mixing step is being carried out. In general, and as known in the art, phase separation into the two phases as described herein may already be visible even upon inspection with the naked eye. However, determination and verification of phase separation may additionally carried out by means well-established in the art. Preferably, the major part of total amount of the at least one fluoroorganic compound as described herein is at least 60 wt.-%, preferably at least 65 wt.-%, and more preferably at least 70 wt.-% of the total amount of fluoroorganic acidic compound initially present in the first solution in step (i). The major part as described herein may even be greater, such as at least 80 wt.-% or even at least 90 wt.-%, based on the total amount of fluoroorganic acidic compound initially present in the first solution in step (i).

Removing the second phase from the first phase in step (iv) may be then carried out by any common means known in the art to the skilled person. Preferably, those means and methods will be selected which are suitable for continuous and/or large-scale operations. Steps (i) to (iv) are repeated at least a second time, wherein the first phase obtained in step (iv) is used again as the solution a. in step (i). In this regard, the process may be carried out again with the first solution consisting essentially of the first phase obtained in step (iv). It is also possible to add the first phase into another fresh portion of first solution a. This may be the case when carrying out the process as described herein in a continuous, large scale operation mode. Accordingly, a more complete removal of the fluoroorganic acidic compound may be achieved.

Since wastewater comprising fluoroorganic compounds often are produced continuously during manufacturing in chemical industry, it is highly desirable that these by-product-containing wastewaters are cleaned from these compounds continuously and not batchwise. Also, the process according to the present disclosure is highly suitable for continuous-mode operations. Accordingly, it is preferred that the process as described herein is carried out in a continuous mode. Preferably, this implies that there is a continuous supply of the solution comprising the at least one fluoroorganic acidic compound (a.) and/or extraction composition (b.) in step (i), a continuous reaction between the fluoroorganic acidic compound in step (ii), continuous separation in step (iii), and/or continuous removal second phase from the first phase in step (iv). Such a continuous mode of operation also preferably implies that each of the process steps as described herein are carried out in different vessels and/or compartments to which the product(s) of the previous product step is transferred into, e.g. by means of respective piping. It is also preferred that the process as described herein is carried out in a continuous mode in that that the second phase is continuously extracting the first phase in a multistage continuous process.

The term "protic solvent" has the meaning commonly used in the art and describes a solvent whose molecules can either donate at least one proton to other molecules. Protic solvents are generally known to the skilled person and may either be selected according to the present needs of the process and the solubility of the fluoroorganic acidic compound. However, it may also occur that the protic solvent is already part of or even constitutes the major part of the wastewater and, therefore, cannot be selected. Preferably, at least one first protic solvent is selected from water, at least one alcohol, at least one acid, and any combinations and mixtures thereof. This has the advantage that a good phase separation between the first phase and the second phase in step (iii) of the process according to the present disclosure may be obtained, which is generally very desirable for a good removal of the second phase from the first phase in subsequent step (iv) and/or the time it takes to complete step (iii). In this regard, it is preferred that the at least one alcohol is selected from methanol, ethanol, propanol, butanol, pentanol, hexanol, and any combinations and mixtures thereof. The acid may be selected from organic acids such as formic acid and acetic acid and from inorganic acids such as H₂SO₄, HCI or HNO₃. In this regard, solutions or dilutions of these acids in water are preferably employed in the process as described herein. The water content of such acid-containing phases may be for example less than 99.9 wt.-%, less than 99 wt.-%, or even less than 98 wt.-%, based on the total weight of the first phase. On the other hand, the content of the alcohols can be up to 99 wt.-%, up to 95 wt.-%, or up to 90 wt.-%, based on the total weight of the first phase.

The at least one fluoroorganic acidic compound may be selected from aliphatic linear, branched or aromatic fluorinated acidic compounds. In this regard, the fluorinated acids may be selected from fluorinated acids comprising a carbon backbone of at least 2 carbon atoms, preferably of at least 3 carbon atoms, for example, at least 5 carbon atoms, wherein the carbon backbone optionally contains at least one oxygen backbone. The fluorinated acids may be selected from fluorinated acids comprising a carbon backbone of 40 carbon atoms or less, preferably of 35 carbon atoms or less, and more preferably of 30 carbon atoms or less. It is particularly preferred that the fluorinated acids are selected from fluorinated acids comprising a carbon backbone of from 3 to 40 carbon atoms, preferably from 4 to 35 carbon atoms, and more preferably from 5 to 30 carbon atoms. The at least one fluorinated acidic compound may comprise at least one carboxylic acid moiety, at least one sulfonic acid moiety, at least one sulfate moiety, and/or at least one alcohol moiety. As known to the skilled person, fluorinated alcohols are acidic and fall under the definition of fluorinated acidic compounds as used herein and may therefore be advantageously removed by the process according to the present disclosure. The fluorinated acids comprise carboxylic acids (R_{f}-SO₃H, sulfates R_{f}-CH₂-O-SO₃⁻), or alcohols (R_{f}-CH₂OH).

The acids/alcohols can be perfluorinated or partially fluorinated. Examples are e.g. CF₃COOH, CF₂HCOOH, C₂F₅COOH, C₂F₄H-COOH, C₄F₉-COOH, C₇F₁₅COOH, CF₃-O-(CF₂)₃-O-CF₂COOH, 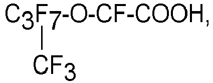 CF₃SO₃H, C₂F₅SO₃H, HOOC-(CF₂)ₙ-CF₂-SO₃H,

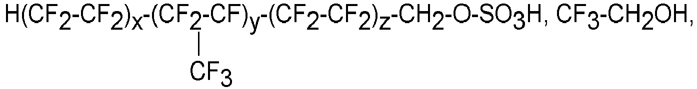

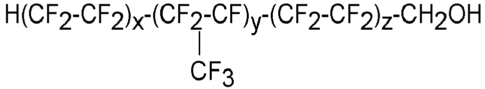

In particular, some fluoroorganic compounds as described herein are known as PFCA's (perfluorinated carboxylic acids). Thus, removal of present commercially available fluoroorganic compounds become feasible with the process according to the present disclosure. In this regard, the fluoroorganic compounds are fluorinated or perfluorinated C2 to C40 carboxylic acids. Typically, the fluoroorganic compounds are present in the first solution in an amount of from 0.4 ppb to 300,000 ppm.

With regard to the at least one alkylamine used in the processes according to the present disclosure, the at least one alkylamine may be selected from primary, secondary and/or tertiary alkylamines, preferably from tertiary alkylamines. The at least one alkylamine comprises linear or branched alkyl groups comprising at least 3 carbon atoms, preferably at least 4 carbon atoms, and more preferably at least 5 carbon atoms. The at least one alkylamine may comprise linear or branched alkyl groups comprising up to 25 carbon atoms, preferably up to 20 carbon atoms, and more preferably up to 18 carbon atoms. Accordingly, it is preferred that the at least one alkylamine comprises linear or branched alkyl groups comprising from 3 to 25 carbon atoms, preferably from 4 to 20 carbon atoms, and more preferably from 5 to 18 carbon atoms. With the at least one alkylamine having carbon atoms in the preferred ranges as described herein, a particularly efficient removal of the fluoroorganic compounds from the first solution as described herein may be achieved.

The amines of the present disclosure may be a liquid or a solid at room temperature. In one embodiment, the amine useful in the present disclose may be described as a water insoluble amine having a solubility in water of less than 20 mg, 10 mg, 5 mg, 3 mg, 2 mg, 1 mg, 0.75 mg, 0.5 mg, or even less than 0.25 mg per 100 mL when measured at ambient conditions.

Preferably, the at least one alkylamine is an amine according to an amine of formula (I):

NR₁R₂R₃ (I)

wherein R₁, R₂, and R₃ may be the same or different and at least one of R₁, R₂, and R₃ is a linear or branched, saturated or unsaturated carbon group, which optionally, may comprise heteroatoms, and optionally, wherein one or two of R₁ to R₃ may be a H. Preferably, any one of R₁, R₂ and R₃ comprise at least 3 carbon atoms, preferably at least 4 carbon atoms, and more preferably at least 5 carbon atoms. For example, the number of carbons in R₁-R₃ can vary, including, for example carbon backbones comprising least 5, 6, 7, 8, 10, 12, 14, 16, 20, or even 24 carbon atoms. Preferably, the at least one alkylamine as used herein is selected from trihexylamine, trioctylamine, tridecylamine, tridocecylamine, alamine, triisooctylamine, trioctyl-decylamine, N-methyldioctylamine, N,N-dimethyloctylamine, tributylamine, octylamine, dioctylamine, and any combinations and mixtures thereof. High purity tertiary amines advantageous for use in the process according to the present disclosure are commercially available, for example, from Cognis, Monheim, Germany under the following trade designations: "ALAMINE 300" (tri-n-octylamine), "ALAMINE 308" (tri-isoctylamine), "ALAMINE 336" (tri(C₈/C₁₀) amine), "ALAMINE 310" (tri-isodecylamine), and "ALAMINE 304" (tri-laurylamine).

The temperature of the mixture, i.e. the aqueous emulsion after addition of the at least one alkylamine, may be controlled in order to advantageously enhance the removal of the fluoroorganic compounds in the process as described herein. In this regard, it is preferred that the process is conducted at a temperature of less than 80 °C, preferably less than 70 °C, more preferably of less than 60 °C. Preferably, the process is conducted at a temperature in the range of from 3 °C to 80 °C, preferably from 5 °C to 70 °C, more preferably from 10 to 60 °C, even more preferred from 15 to 50 °C. In particular, the process as described herein may be carried out at room temperature and general ambient conditions. This is very advantageous for carrying out the process on an industrial scale since neither cooling nor heating is required, which greatly helps to save on energy, equipment and resources and makes the process as described herein very advantageous from both economic and ecologic points of view.

Generally, the at least one alkylamine is present in at least one organic solvent. This has the advantage that the extraction of the at least one fluoroorganic compound may be sped up. In this regard, it is preferred that the at least one organic solvent is not miscible with water. This has the effect that a clear separation between an aqueous phase and an organic phase may be obtained. Generally, the at least one organic solvent has a solubility in water at a temperature of 20 °C according to the method of the "Prüfmethoden-Verordnung (EG) Nr. 440/2008, Teil A, Methode A.6 (Test according to OECD-Prüfrichtlinie 105), of less than 500 ppm and more preferably less than 50 ppm. Furthermore, i the at least one organic solvent exhibits a boiling point of at least 100 °C, preferably of at least 125 °C, and more preferably of at least 150 °C. The use of organic solvents exhibiting boiling points at ambient pressure above the aforementioned temperatures, i.e. high-boiling organic solvents, has the advantage of an improved inherent process safety in that the risk of solvent evaporating or even boiling off at the preferred temperatures of the process as described herein is greatly reduced. Preferably, the at least one organic solvent is selected from linear hydrocarbons, cyclic hydrocarbons, ethers, aromatic solvents, and any combinations and mixtures therefrom. As an alternative or in addition to the beforementioned solvents, it is preferred to use fluorinated solvents, e.g. HFE's, Novec-Fluids (available from 3M). Such solvents preferably comprise perfluorinated trialkylamines (NR₃) and/or fluorinated ethers (e.g. Methoxyperfluorobutene HFE 7100). It is preferred that the boiling point of fluorinated solvents is higher than 50 °C. The non-fluorinated solvent should have no functional group, e.g. ester, ketone. Furthermore, it is preferred that the volume ratio of solvent to amine is from 1 : 1 to 1000 : 1. In this regard, it is also preferred that the molar ratio of amine to fluorinated acid/alcohol is at least 1 : 1 up to 1000 : 1, preferably from 1 : 2 up to 100 : 1. These solvents may also be halogenated such as chlorinated or fluorinated, which is advantageous in extracting the fluorinated or perfluorinated compounds in the process according to the present disclosure. In this regard, it is preferred that the at least one organic solvent is selected from toluene, xylene, mesitylene, octane, and any combinations and mixtures or halogenated derivates therefrom.

Contacting the first solution with the second solution comprising the at least one alkylamine in step (i) of the process as described herein, preferably present in at least one organic solvent as described herein, may be carried out by adding the second solution to or into the first. After addition, it is preferred that the resulting mixture is agitated for at least the amount of time sufficient to contact the at least one alkylamine with the fluoroorganic compound present in the first solution. As a general rule, however, the contact time between the at least one alkylamine, i.e. the second solution and the first solution is preferably in excess of 0.1 seconds with some equipment, but generally less than 5, 4, 3, or even 2 hours. Exemplary equipment that may be used for agitation include: vortexers, agitators, sonicators, and other such equipment as known in the art.

Without wanting to be bound by theory, it is believed that the extraction of the fluoroorganic acidic compounds, in particular acids, from the first solution of protic solvents with alkylamines as described herein, is effectuated with the fluoroorganic acid and the alkylamine forming an ion pair hydrophobic organic liquid. This would then separate from the protic solvent of the first solution and allow subsequent for further extraction and treatment. This separation is even enhanced when using the least one organic solvent in the second solution as described herein when the first solution is contacted with the at least one alkylamine in the process according to the present disclosure. Because of this immiscibility, the first phase (i.e. protic) phase and the second phase (i.e. organic phase or non-protic phase) generally separate after agitation has stopped. If, however, the mixture, although bi-phasic, does not phase separate on its own, measures as known in the art, may be used to promote the phase separation, including centrifugation. Separation of the first phase from the second phase in step (iv) of the process as described herein may be carried out by any appropriate means known in the art, including, preferably, phase separation. Surprisingly, by the process according to the present disclosure, fluorinated acids can be separated from other acids (e.g. CH₃COOH vs. CF₃COOH).

The process according to the present disclosure is preferably carried out in a continuous mode. This is very advantageous for applications in industrial scale, where e.g. continuous treatment of wastewater is necessary. Preferably, the process as described herein is carried out in extraction columns, preferably multi-staged extraction columns. In this regard, it is preferred that multi-staged extraction columns as known in the art comprise at least two stages. Preferably, the process is carried out in Scheibel-columns, centrifugal extractors (e.g. from Robatel), and other means known to the skilled person in the art.

It was further found that the processes according to the present disclosure show best results when carried out at certain pH-values. In particular, it was found that the best results were obtained at low pH-values, while medium pH-values showed only mediocre results and high pH-values failed to achieve effective removal of fluoroorganic compounds. Running the process as described herein at the preferred pH-values may be easily achieved by adjusting the pH-value of the first solution (a.) before contacting it with the at least one alkylamine. Accordingly, it is preferred that the pH-value of the first solution is adjusted to a pH-value of up to 5, preferably of up to 4, more preferably of up to 3 before contacting with at least one alkylamine in step (i). Also, this regeneration process is preferably carried out as continuous, multistage process.

In order to run a process in the chemical industry in an economically efficient way and in order to comply with environmental benign requirements, there is a desire in the art to recollect and, in if feasible, recycle or reuse the material and compounds used in the process. In the process according to the present disclosure, it is desirable to at least recollect the at least one alkylamine used in the process. It was found that this may be achieved by adding at least one base to the organic phase after carrying out the previously described process steps. Hence, it is preferred that the process according to the present disclosure comprises an additional step (vi) of adding at least one base to the organic phase in order to regenerate the at least one alkylamine. Carrying out this additional step has the advantage that the at least one alkylamine is regenerated from its protonated form and can be recollected and/or recycled into the processes as described herein. Therefore, it is preferred that step (vi) is carried out continuously. It is further preferred that the regenerated at least one alkylamine is recycled into use in step (i) of the process as described herein. Adding a base and recollecting the regenerated alkylamine may be carried out by common means known to the skilled person. With regard to the base, it is preferred that the at least one based is selected from KOH, NH₄OH, NaOH, and any combinations and mixtures thereof. These bases provide favourable results, dissolve readily in aqueous media, and are commercially available in any given amounts at reasonable prices.

The process according to the present disclosure is preferably a continuous process for extracting fluoroorganic acidic compounds from a solvent stream, preferably a wastewater stream. The water stream is preferably selected from an industry sewage water stream, a manufacturing wastewater stream, or a water stream from a vessel comprising a solvent phase containing at least one fluoroorganic acidic compound.

### Examples

The present disclosure is further described without however wanting to limit the disclosure thereto. The following examples are provided to illustrate certain embodiments but are not meant to be limited in any way. Prior to that some test methods used to characterize materials and their properties will be described. All parts and percentages are by weight unless otherwise indicated.

### Test Methods

### NMR Analytical methods:

### (1) Water Phase Samples:

For each sample, an aliquot of fluid was accurately weighed into a 5 mm o.d. glass NMR tube, was spiked with small accurately weighed amount of 2,2,2-trifluoroethanol internal standard, and was diluted with a small amount of deuterium oxide (D₂O) for NMR analyses. 600 MHz ¹H-NMR and 565 MHz ¹⁹F-NMR spectra were acquired. The trifluoroethanol was used in all cases as the internal standard to permit the calculation of the absolute weight percent concentrations of sample components without needing to identity and quantify all of the other components in the samples.

### (2) Organic Phase Samples:

For each sample, an aliquot of fluid was accurately weighed into a 5 mm o.d. glass NMR tube, was spiked with small accurately weighed amount of 1,4-bis(trifluoromethyl)benzene (p-HFX) internal standard, and was diluted with a small amount of deuterium acetonitrile (CD₃CN) for NMR analyses. 600 MHz ¹H-NMR and 565 MHz ¹⁹F-NMR spectra were acquired. The p-HFX was used in all cases as the internal standard to permit the calculation of the absolute weight percent concentrations of sample components without needing to identity and quantify all of the other components in the samples.

### Gas Chromatography Analysis

1 mL of the aqueous sample and 0.6 mL of BF₃/MeOH-complex solution (20%, obtained from Merck) are filled in 10 mL head-space vial and intermediately closed. After shaking, the vial is heated to 70 °C for 30 min using the autosampler unit of the headspace GC-instrument and afterwards analyzed by headspace-GC-FID with external calibration.

### Liquid Chromatography - mass spectrometrie (LC-MS):

The submitted samples were dissolved in methanol prior to analysis. Standard solutions were prepared from an aqueous solution of Adona that was determined to have a concentration of 28.36% by NMR.

| | |
|---|---|
| Instrument: | Agilent 6470 Triple Quad LCMS MAID 1665 |
| Column: | Zorbax Bonus RP, 4.6 x 75 mm, 3.5 µm |
| Solvent A: | Water with 6 mM ammonium acetate |
| Solvent B: | Methanol with 6 mM ammonium acetate |
| Gradient: | 75% B to 100% B at 4 minutes |
| Injection: | 2 µL |
| Col. Temp: | 40°C |
| Flow Rate: | 0.5 mL/min |
| MS: | Negative electrospray |

### Materials used:

| | |
|---|---|
| Trioctylamine | obtained from Sigma Aldrich (99.7% purity) |
| Trifluoroacetic acid | Obtained from Sigma Aldrich (97% purity) |
| Pentafluoropropionic acid | Obtained from Sigma Aldrich (97% purity) |
| Tetrafluoropropionic acid | Obtained from abcr (94% purity) |
| Alamine 336 | Obtained from Cognis (>95% purity) |
| Mesitylene | Obtained from Sigma Aldrich (98% purity) |
| Octane | Obtained from Sigma Aldrich (98% purity) |
| KOH (Potassium hydroxide) | Obtained from Sigma Aldrich (>85% purity) |
| ADONA | Emulsifier, chemical formula CF3O-(CF2)3-O-CHF-CF2-COONH4, available from Anles Ltd., St. Petersburg, Russia |
| AOPA | emulsifier, chemical formula CF3-O-(CF2)2-COONH4, prepared as described in US 6,482,979 A1 |

### Example 1 (Comparative Example):

200 mL of an aqueous solution containing 328 ppm trifluoroacetic acid (TFA) and 38 ppm pentafluoropropionic acid is filled in a 250 mL plastic bottle. After adjusting the pH value of the aqueous solution to the target value (pH 3) with sulfuric acid, the extracting agent solution (1 g Alamine 336 dissolved in 19 g mesitylene) is added. Upon tightly sealing the bottle, the obtained mixture is vigorously shaken for 1 minute. After shaking the solution, the aqueous phase is separated from the extracting agent solution by filling the mixture in a separation funnel for phase splitting. NMR on the aqueous phase revealed a significant reduction of TFA down to 27 ppm and pentafluoropropionic acid <1 ppm. All steps were performed at room temperature (20 °C).

### Example 2:

The extraction described in example 1 is repeated 4 times (4 stage extraction) on the similar aqueous solution as in example 1 in the following procedure. After each extraction step (1^{st} extraction similar procedure as example 1), the aqueous phase is separated from the extracting agent (1 g Alamine in 19 g mesitylene) and filled in a new 250 mL plastic bottle. Then an unused (new) extracting agent solution (1 g Alamine in 19 g mesitylene) is added, the mixture is shaken and again, the aqueous phase is separated. This procedure is repeated 3 times, so that the aqueous solution is purified in 4 stages (extraction steps). NMR on the aqueous solution after four extraction steps revealed a drastic reduction of TFA down to 0.3 ppm and pentafluoropropionic acid <1ppm.

### Example 3

The procedure as described in example 2 (a 4-stage batch extraction) was followed. The experiments demonstrate the benefit of the multistage extraction and the differences in solvents (Mesitylene/Octane). The results are listed below:

| | **initial conc. ppm** | **Mesitylene** | | | **Octane** | |
|---|---|---|---|---|---|---|
| | | **1. stage ppm** | **2. stage ppm** | **4. stage ppm** | **2. stage ppm** | **4. stage ppm** |
| CF₃-CO₂ | 330 | 43 | 5 | 0.3 | 50 | 14 |
| CF₃CFH-CO₂ | 230 | 58 | 12 | <1 | 73 | 29 |
| CF₃CF₂-CO₂ | 38 | <1 | <1 | <1 | <1 | <1 |

### Example 4

A 4-stage centrifugal extractor LX-204 from Rousselet Robatel (France) was used to purify a water stream with different fluorinated species listed in the table below.

| | |
|---|---|
| Parameters: | Phase ratio Alamine (5 %) in Mesitylene vs. water = 0.1 w/w; flow rate: 4.1 kg/min water phase, 0.41 kg/min organic phase; RPM = 3300; RT; |
| | pH of water phase: 1.7. |

Results:

| | **clear phase splitting** | | | |
|---|---|---|---|---|
| | **initial conc. ppm** | **4. stage ppm** | **Organic phase ppm** | **Recovery organic phase %** |
| -CF₃-CO₂ | 128 | <1 | 1130 | 87.9 |
| -CF₃CFH-CO₂ | 114 | <2 | 791 | 69.4 |
| -CF₃CF₂-CO₂ | 32 | <2 | 265 | 82.9 |

### Regeneration of organic Phase

The collected organic phase was treated in the same apparatus LX-204 with 20 w% KOH at RT.

| | |
|---|---|
| Parameters: | Phase Ratio Organic /aqueous KOH = 20 w/w. flow rates: 0.09 kg/min aqueous KOH phase, 1.82 kg/min organic phase, RPM 3000. |

Results: All fluorinated species were below detection limit (<1 ppm). The organic Alamine phase can be reused for further extractions.
The % recovery for the organic phase after regeneration is based on calculation with flow rates.

### Example 5

This example shows that even a highly loaded water stream can be extracted.
A mixture of 3 wt.-% CF₃COOH containing high fractions of other non-fluorinated acids (65 wt.-% acetic acid, 10 wt.-% HF, 2.5 wt.-% HNO₃) in water treated with Alamine/Mesitylene as described in Example 1 with the exception that the Alamine/ CF₃COOH ratio was varied. Sample 1 uses the pure acidic solution. In sample 2, the initial 3 wt.-% CF₃COOH solution was diluted 1:1 with deionized water.

The results are:

| Initial CF₃COO-conc. | Trioctylamine/CF₃COO-Ratio | CF₃COO concentration after 1^{st} extraction | CF₃COO concentration after 4 extractions |
|---|---|---|---|
| 3 wt.-% | 1.6 | 0.9 wt.-% | 0.04 wt.-% |
| 1.5 wt.-% | 2.6 | 0.2 wt.-% | 10 ppm |

### Example 6

33 wt.-% aqueous HCl containing 240 ppm CF₃COOH was two times extracted with Trioctylamine/Mesitylene (weight ratio 1:19). After the first extraction step the level of TFA was 70 ppm. After the second extraction step the TFA-content was 25 ppm.

## Claims

1. A process for removing fluoroorganic acidic compounds from a solution comprising at least one protic solvent, comprising the following steps:
(i) forming a mixture of
a. a solution comprising at least one fluoroorganic acidic compound comprising less than 10 carbon atoms and at least one protic solvent, wherein the solution has a pH-value of up to 6, with
b. an extraction composition comprising at least one alkylamine and at least one organic solvent;
(ii) reacting the fluoroorganic acidic compound with the alkylamine to form a hydrophobic ionic compound comprising the anion of the fluoroorganic acidic compound and the cation of the alkyl amine;
(iii) separating the mixture into a first phase comprising the at least one protic solvent and no greater than 50% by weight of the total amount of the at least one fluoroorganic acidic compound initially present in the solution in step (i); and a second phase comprising the at least one organic solvent and the hydrophobic ionic compound;
(iv) removing the second phase from the first phase; and
(v) repeating steps (i) to (iv) at least a second time, wherein the first phase obtained in step (iv) is used as the solution in step (i).

2. The process according to claim 1, wherein the process is carried out in a continuous operation mode.

3. The process according to claim 1 or claim 2, wherein the process is carried out in a continuous operation mode in that the second phase is continuously extracting the first phase in a multistage continuous process.

4. The process according to any one of the preceding claims, wherein the at least one first protic solvent is selected from water, at least one alcohol, at least one acid, and any combinations and mixtures thereof.

5. The process according to any one of the preceding claims, wherein the pH-value of the first phase is up to 5, preferably up to 4, and more preferably up to 3.

6. The process according to any one of the preceding claims, wherein the at least one fluoroorganic acidic compound is selected from aliphatic linear, branched or aromatic fluorinated acidic compounds, preferably wherein the at least one fluorinated acidic compounds comprises at least one carboxylic acid moiety, at least one sulfonic acid moiety, at least one sulfate moiety, and/or at least one alcohol moiety.

7. The process according to any one of the preceding claims, wherein the at least one alkylamine is selected from primary, secondary and/or tertiary alkylamines, preferably from tertiary alkylamines, preferably wherein the at least one alkylamine comprises linear or branched alkyl groups comprising at least 3 carbon atoms, preferably at least 4 carbon atoms, and more preferably at least 5 carbon atoms.

8. The process according to any one of the preceding claims, wherein the at least one alkylamine is an amine according to an amine of formula (I):
NR₁R₂R₃ (I)
wherein R₁, R₂, and R₃ may be the same or different and at least one of R₁, R₂, and R₃ is a linear or branched, saturated or unsaturated carbon group, which optionally, may comprise heteroatoms, and optionally, wherein one or two of R₁-R₃ may be a H.

9. The process according to any one of the preceding claims, wherein the the major part of total amount of the at least one fluoroorganic acidic compound is at least 60 wt.-%, preferably at least 65 wt.-%, and more preferably at least 70 wt.-% of the total amount of fluoroorganic acidic compound initially present in the solution a. in step (i)..

10. The process according to any one of the preceding claims, wherein the at least one organic solvent has a solubility in water at 20 °C of less than 0.1 %, preferably less than 500 ppm and more preferably less than 50 ppm.

11. The process according to any one of the preceding claims, wherein the process is carried out in extraction columns, preferably multi-staged extraction columns, Scheibel columns, centrifugal extractors, and any combinations thereof.

12. The process according to any one of the preceding claims, wherein the process comprises an additional step (vi) of adding at least one base to the organic phase in order to regenerate the at least one alkylamine.

13. The process according to claim 12, wherein step (vi) is carried out continuously and/or as a multistage process.

14. The process according to claim 12 or claim 13, wherein the regenerated at least one alkylamine is recycled into use in step (i).

15. The process according to any one of the preceding claims, wherein the process is a continuous process for extracting fluorinated compounds from a solvent stream, preferably a water stream.
